# EUROPÄISCHE PATENTANMELDUNG

(11) **EP 2 380 485 A1**
(43) Veröffentlichungstag der Anmeldung: **26.10.2011**
(21) Anmeldenummer: 11003219.0
(22) Anmeldetag: 16.04.2011
(51) Int. Cl.: A61B 1/00

(54) **Starres Endoskop**

(30) Priorität: 21.04.2010 DE 102010017880
(71) Anmelder: Richard Wolf GmbH, 75438 Knittlingen (DE)
(72) Erfinder: Körner, Eberhard, Dipl.-Ing., 75438 Knittlingen (DE); Boebel, Manfred, 75245 Bauschlott (DE); Scholl, Waldemar, B. Eng., 76698 Ubstadt-Weiher (DE)
(74) Vertreter: Vollmann, Heiko

(57) **Zusammenfassung**

Das starre Endoskop weist einen schaftförmigen Mittelteil (8) auf, an dem proximalseitig ein Handhabenteil (9) anschließt und der distalseitig ein Distalteil (10) mit einem Optikfenster (14) aufweist. Der Handhabenteil (9) ist versetzt oder schräg zur Längsachse (13) des Mittelteils (8) angeordnet, ebenso der Distalteil (10), um im Bereich der Handhabe des Arbeitsinstrumentes sowie distalseitig des Werkzeuges Freiraum zu schaffen.

## Beschreibung

Die Erfindung betrifft ein starres Endoskop mit den im Oberbegriff des Anspruchs 1 angegebenen Merkmalen.

Ein solches starres Endoskop weist typischerweise einen schaftförmigen Mittelteil auf, an den proximalseitig ein Handhabenteil und distalseitig ein Distalteil anschließen, wobei im Distalteil ein Optikfenster vorgesehen ist, welches bei einem aus ausschließlich aus optischen Bauelementen aufgebauten optischen System das Ausblickfenster und bei einem elektronischen/optischen System das Fenster für den optischen Strahlengang bildet. Derartige starre Endoskope können entweder als reine Endoskopoptik oder aber mit einem oder mehreren Arbeits-/Saug-/Spülkanälen ausgestattet sein, über die ein oder mehrere Instrumente einführbar sind.

Ein solches starres Endoskop in Form eines Laparoskopes wird von der Richard Wolf GmbH unter der Typennummer 8915.402 angeboten. Es weist einen Handhabenteil auf, an dessen Ende ein Okular der Endoskopoptik angebracht ist, das schräg zur Längsachse des schaftförmigen Mittelteils des Instrumentes angeordnet ist und somit den Zugang für einen fluchtend zum Mittelteil mündenden Arbeitskanal mit genügend Freiraum belässt. Auch zählt es zum Stand der Technik, das vorbeschriebene starre Endoskop mit einem versetzt zum schaftförmigen Mittelteil angeordneten Handhabenteil herzustellen, so dass der Einblick in die Endoskopoptik in Richtung der Längsachse des schaftförmigen Mittelteils, jedoch versetzt dazu erfolgt.

Bei der laparoskopischen Singleporttechnik wird anstelle von mehreren Trokarhülsen mit einer einzigen, speziell angepassten Trokarhülse gearbeitet. Durch diese Trokarhülse wird nicht nur eine Optik, also ein starres Endoskop der in Rede stehenden Art, sondern zusätzlich weitere Arbeitsinstrumente in den Bauchraum eingeführt. Kritisch dabei ist, dass die Instrumente dabei miteinander in Konflikt geraten können, wodurch das Arbeiten erschwert wird und zum Beispiel überkreuz gearbeitet werden muss.

Proximalseitig kann dieses Problem grundsätzlich durch eine Verlängerung im Griffbereich vermindert werden, was jedoch die Instrumente nicht nur unhandlicher macht, sondern auch störanfälliger und teurer. Distalseitig Abhilfe schaffen flexible Endoskope . Diese bieten deutlich mehr Freiheitsgrade, können aber die Konfliktgefahr im Bauchinneren nicht mindern, da das distale Endoskopende gegenüber den Instrumenten schräg gestellt werden muss. Hinzu kommt, dass derartige flexible Endoskope deutlich aufwändiger, störanfälliger und schwieriger in der Handhabung sind als starre Endoskope.

Vor diesem Hintergrund liegt der Erfindung die Aufgabe zugrunde, ein gattungsgemäßes Endoskop so auszubilden, dass es insbesondere zum Einsatz in der Singleporttechnik sowie auch bei anderen endoskopischen Verfahren vorteilhaft einsetzbar ist und die vorgenannten Nachteile und Probleme zumindest vermindert.

Diese Aufgabe wird gemäß der Erfindung durch ein starres Endoskop mit den in Anspruch 1 angegebenen Merkmalen gelöst. Vorteilhafte Ausgestaltungen der Erfindung sind in den Unteransprüchen sowie der nachfolgenden Beschreibung angegeben.

Das erfindungsgemäße starre Endoskop weist einen schaftförmigen Mittelteil auf, an dem proximalseitig ein Handhabenteil anschließt und der distalseitig ein Distalteil mit einem Optikfenster aufweist, wobei der Handhabenteil versetzt oder schräg zur Längsachse des Mittelteils angeordnet ist. Erfindungsgemäß ist auch der Distalteil versetzt oder schräg zur Längsachse des Mittelteils angeordnet.

Grundgedanke der vorliegenden Erfindung ist es, lediglich den schmalen langgestreckten schaftförmigen Mittelteil in Flucht zur Trokarhülse, welche den Zugang in den Bauchraum bildet, anzuordnen und im Übrigen sowohl den Handhabenteil als auch den Distalteil versetzt zur Längsachse anzuordnen, damit nicht nur handhabenseitig, sondern auch bauchraumseitig eine Instrumentenkollision bzw. eine Behinderung der endoskopischen Instrumente untereinander vermieden wird.

Das erfindungsgemäße starre Endoskop kann dabei entweder als Einzeloptik ausgebildet sein oder auch ein oder mehrere Arbeit-/Saug-/Spülkanäle aufweisen. Durch die versetzte bzw. schräge Anordnung des Distalteils wird konstruktiv dafür gesorgt, dass zwar einerseits die Betrachtung des Operationsfeldes innerhalb des Körperraumes und insbesondere auch das distale Ende des oder der Arbeitsinstrumente sichtbar ist, jedoch das Distalteil selbst die Handhabung des oder der Arbeitsinstrumente nicht behindert.

Als besonders vorteilhaft hat es sich erwiesen, Handhabenteil und Distalteil bezogen auf die Längsachse des Mittelteils fluchtend anzuordnen, d.h. die Abkröpfung oder Schrägstellung in derselben radialen Ebene anzuordnen. Eine Handhabung eines so ausgebildeten Endoskops ist in der Praxis besonders einfach, da der innenliegende, im Einsatz nicht sichtbare Versatz des Distalteils bzw. dessen Schrägstellung winkelmäßig genauso angeordnet ist, wie der proximalseitige sichtbare Teil. Dies erleichtert die räumliche Zuordnung während des Eingriffs. Unter schräg zur Längsachse des Mittelteil angeordnet im Sinne der vorliegenden Erfindung ist auch eine bogenförmige Anordnung zu verstehen, bei der das Distalteil vom Mittelteil ausgehend nicht nur zunächst schräg radial nach außen, sondern bogenförmig und im Verlauf nachfolgend wieder nach innen oder in einer anderswie geeigneten Kurvenform verläuft. Dabei hat ein bogenförmiger Verlauf, bei dem das Distalteil zunächst von der Längsachse des schaftförmigen Mittelteils nach außen und dann wiederum nach innen gerichtet wird, wobei es jedoch mit Abstand zur Längsachse endet, den Vorteil, dass eine Geradblickoptik verwendet werden kann. Alternativ kann das Distalteil S-förmigen oder einen anderen für das jeweilige Operationsgebiet besonders zweckmäßigen Kurvenverlauf aufweisen. Dabei ist es von Vorteil, wenn das Distalteil oder zumindest ein Abschnitt davon ebenfalls schaftförmig ausgebildet ist, derart, dass es den schaftförmigen Mittelteil vorzugsweise mit gleichbleibendem Querschnitt fortsetzt. Dann ergibt sich ein insbesondere distalseitig besonders schlankes Instrument, das vorteilhaft mit einer konventionellen Optik ausgestattet werden kann, damit eine optische Verbindung zwischen Distalteil und Handhabenteil besteht.

Alternativ kann das Distalteil gegenüber dem Mittelteil gemäß einer Weiterbildung der Erfindung aufweitend ausgebildet sein und einen elektronischen Bildsensor aufweisen, der optisch mit dem Optikfenster verbunden ist und dessen elektrische Anschlüsse mittelbar oder unmittelbar zum Handhabenteil geführt und dort herausgeführt sind. Diese Konstruktion kann dazu genutzt werden, den Mittelteil des Endoskops besonders schlank zu halten, da in diesem Bereich dann lediglich Kabeldurchführungen verlaufen und kein optisches System. Am Handhabenteil sind dann lediglich die elektrischen Anschlüsse herauszuführen, entweder in Form eines Anschlusssteckers oder in Form einer fest angebundenen elektrischen Leitung. Dabei können nur der Bildsensor im Distalteil und die Auswertelektronik im Handhabenteil oder außerhalb des Endoskops angeordnet sein, dann wird der elektronische Bildsensor unmittelbar mit seinen elektrischen Anschlüssen zum Handhabenteil geführt und dort herausgeführt. Wenn jedoch die Auswertelektronik oder ein Teil der Auswertelektronik im Distalteil zusammen mit dem Bildsensor angeordnet sind, dann sind die elektrischen Anschlüsse des Bildsensors mittelbar, also über die Anschlüsse der Bildverarbeitungselektronik zum Handhabenteil geführt und dort herausgeführt. Optisch ist der Bildsensor mit dem Optikfenster verbunden, um auf diese Weise mittels des Sensors, der den Teil einer Videokamera bildet, Übersicht über das Operartionsgebiet innerhalb des Hohlraumes bzw. die dort handzuhabenden Endeffektoren des Arbeitsinstrumentes zu bekommen.

Während bei rein optischer Ausbildung des optischen Systems am Handhabenteil typischerweise ein Okular und/oder Optikanschluss vorgesehen sind, an die z.B. ein Videokamerakopf anschließbar ist, kann bei Ausbildung des Endoskops mit einem elektronischen Bildsensor proximalseitig die Betrachtung über einen externen Bildschirm erfolgen .

Das Optikfenster des erfindungsgemäßen Endoskops ist vorteilhaft am distalen Ende des Distalteils vorgesehen, und zwar vorzugsweise schräg zur Längsachse des Distalteils angeordnet. Über die Schräganordnung des Fensters kann das Blickfeld gezielt auf den hier interessierenden Endeffektorenbereich gerichtet werden, ohne dass insbesondere ein optisch konstruktiv aufwändiger Aufbau erforderlich ist. Alternativ kann das Optikfenster auch in einer Umfangswand des Distalteils angeordnet sein. Eine solche Anordnung wird insbesondere dann zweckmäßig sein, wenn das Distalteil gegenüber dem schaftförmigen Mittelteil schräg nach außen verlaufend angeordnet ist.

Bei dem erfindungsgemäßen starren Endoskop sind vorteilhaft Beleuchtungsmittel integriert, vorzugsweise mit mindestens einer Leuchtdiode arbeitende Beleuchtungsmittel. Diese können entweder im Distalteil oder im Handhabenteil vorgesehen sein. Besonders vorteilhaft ist die Anordnung im Distalteil, da dann keine Lichtleichter durch den schaftförmigen Mittelteil geführt werden müssen.

Gemäß einer vorteilhaften Weiterbildung des erfindungsgemäßen Endoskops weist der schaftförmige Mittelteil keinen runden, sondern einen ovalen Querschnitt auf, um im Bereich der Trokarhülse mehr Platz für die Arbeitsinstrumente zu schaffen. Dabei ist die Querschnittsanordnung vorzugsweise derart, dass der Versatz oder die Schrägstellung des Distalteils und/oder des Handhabenteils in Richtung der kurzen Querschnittsachse angeordnet sind. Der Querschnitt liegt also mit seinem großen Biegeradius, also mit seiner flachen Seite an der Trokarhülse an und ragt gegenüber einem runden Querschnitt deutlich weniger in diese hinein.

Die Erfindung ist nachfolgend anhand von in der Zeichnung dargestellten Ausführungsbeispielen dargestellt. Es zeigen:
- Figur 1 -: in stark vereinfachter schematischer Darstellung eine Trokarhülse mit einem durchgeführt starren Endoskop gemäß der Erfindung sowie mit einem Arbeitsinstrument,
- Figuren 2a - 2c-: drei Ausführungen des Distalteils des Endoskops,
- Figuren 3a und 3b-: zwei Ausführungsvarianten des Handhabenteils des Endoskops und
- Figuren 4a und 4b-: zwei Ausführungsvarianten betreffend den Schaftquerschnitt des Endoskops.

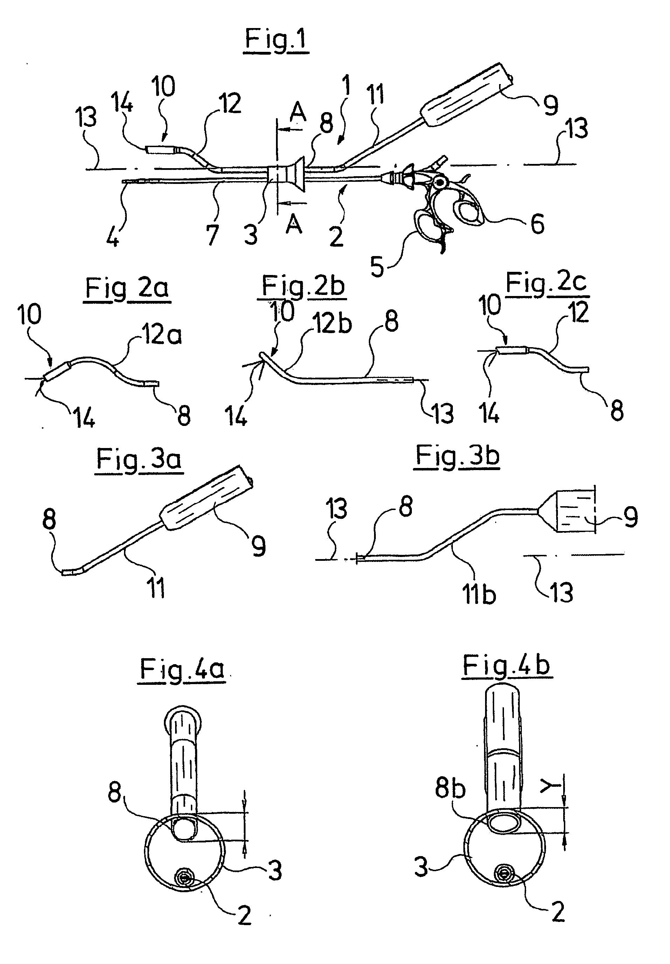

Anhand von Figur 1 ist die Anordnung eines starren Endoskops 1 mit einem Arbeitsinstrument 2 innerhalb einer Trokarhülse 3 dargestellt. Die Trokarhülse 3, die den Zugang zur Körperhöhle aufrechterhält, ist im Wesentlichen zylindrisch ausgebildet und proximalseitig trichterförmig aufgeweitet. Dabei liegt der trichterförmig aufgeweitete Bereich außerhalb des Körperinneren, wohingegen der zylindrische Bereich die Haut und die Bauchdecke durchdringt und somit einen Zugang ins Körperinnere bildet.

Bei dem Arbeitsinstrument 2 handelt es sich um eine Zange, deren distalseitige Endeffektoren 4 mittels zwei proximalseitig angeordneten Griffteilen 5, 6 zu handhaben sind. Verbunden sind die Effektoren 4 und die Griffteile 5, 6 über einen graden Schaftteil 7.

Das Endoskop, das parallel zu dem Schaftteil 7 durch die Trokarhülse 3 hindurchgeführt ist, weist einen schaftförmigen Mittelteil 8 auf, der gradlinig ausgebildet und etwa parallel zum Schaftteil 7 geführt ist. An den schaftförmigen Mittelteil 8 schließen proximalseitig ein Handhabenteil 9 an sowie distalseitig ein Distalteil 10. Dabei ist der Handhabenteil 9 schräg zur Längsachse 13 des schaftförmigen Mittelteils 8 angeordnet und der Distalteil 10 versetzt dazu.

Bei der Ausführung gemäß Figur 1 schließt an den schaftförmigen Mittelteil 8 ein dem gegenüber schräg nach außen abgebogener proximaler Schaftbereich 11 an, über den der eigentliche Handhabenteil 9, der typischerweise an seinem proximalen Ende ein nicht dargestelltes Okular aufweist, angeschlossen ist. Ein distaler Schaftbereich 12 verbindet den schaftförmigen Mittelteil 8 mit dem eigentlichen Distalteil 10, der bei der anhand den Figuren 1, 2a und 2c dargestellten Ausführungsvarianten gegenüber dem Schaftbereich 12 aufgeweitet ausgebildet ist und eine im Wesentlichen zylindrische Form aufweist.

Wie die Figur 1 deutlich zeigt, sind sowohl Handhabenteil 9 als auch Distalteil 10 mit deutlichem Abstand zu dem Arbeitsinstrument 2 in den jeweiligen Bereichen angeordnet, so dass sich die Instrumente weder außen (proximalseitig) noch innen (distalseitig) behindern. Der anhand von Figur 1 dargestellte Distalteil 10 ist über einen parallel versetzten distalen Schaftbereich 12 an den schaftförmigen Mittelteil 8 angebunden, so dass der Distalteil 10 parallel und mit Abstand zu den Endeffektoren 4 angeordnet ist. Der proximale Schaftbereich 11 hingegen, ist gegenüber dem schaftförmigen Mittelteil 8 schräg nach außen abgebogen, entfernt sich also mit zunehmendem proximalen Abstand von der Trokarhülse 3 radial von der Längsachse 13 des schaftförmigen Mittelteils 8.

Anhand der Figuren 2a, 2b und 2c sind alternative Ausgestaltungen des Distalteils 10 und des distalen Schaftbereichs 12 dargestellt. Bei der Ausführungsvariante gemäß Figur 2a hat der distale Schaftbereich 12a einen etwa S-förmigen Verlauf, so dass sich der Schaft dort ausgehend von dem schaftförmigen Mittelteil 8 distalwärts zunächst von der Längsachse 13 entfernt, um dann in einer Kurve wieder auf diese zurückgeführt zu werden. Am distalen Ende des Schaftbereichs 12a ist der Distalteil 10 angeordnet, dessen Optikfenster 14 in Richtung schräg auf die Längsachse 13 weist und somit eine Beobachtung der Endeffektoren 4 bzw. des davon umgebenden Operationsgebietes ermöglicht.

Bei der Ausführung gemäß Figur 2b geht der schaftförmige Mittelteil 8 in den distalen Schaftbereich 12b über, der gegenüber der Längsachse 13 schräg verläuft. Das Optikfenster 14 ist bei dieser Ausführung im Außenumfang des distalen Schaftbereiches 12 angeordnet, der zugleich das Distalteil des Endoskops bildet.

Anhand von Figur 2c ist die Ausführungsvariante aus Figur 1 dargestellt, bei welcher der distale Schaftbereich 12 abgesetzt, d.h. gekröpft ausgebildet ist und distalseitig einen zylindrischen Distalteil 10 aufnimmt, an dessen distalen Ende das Optikfenster 14 schräg zur Längsachse des Distalteils 10 angeordnet ist, um auf diese Weise das Arbeitsgebiet der Endeffektoren 4 zu erfassen.

Anhand der Figuren 3a und 3b ist dargestellt, in welcher Weise der proximale Schaftbereich 11 mit dem Handhabenteil 9 an den schaftförmigen Mittelteil 8 angebunden sein kann. Die Ausführung gemäß 3a entspricht der anhand von Figur 1 dargestellten und beschriebenen. Alternativ dazu ist bei der Ausführung gemäß Figur 3b der proximale Schaftbereich 11 b abgesetzt, d.h. gekröpft ausgebildet, so dass der Handhabenteil 9 parallel versetzt zur Längsachse 13 des schaftförmigen Mittelteils 8 angeordnet ist.

Der Handhabenteil 9 ist in den Figuren nur beispielhaft durch einen zylinderförmigen Körper dargestellt, es versteht sich, dass dieser durch einen Optikanschluss gebildet sein, ein Okular aufweisen, oder in sonstiger geeigneter Weise ausgebildet sein kann. Die hier dargestellte Form soll lediglich ein Beispiel für jede übliche oder geeignete Form eines Handhabenteils einer Endoskopoptik stehen.

Anhand der Figuren 4a und 4b sind zwei Alternativen der Querschnittsform des schaftförmigen Mittelteils 8 dargestellt. Die Ausführung 4a, die einen Schnitt der Trokarhülse 3 im Bereich der in Figur 1 dargestellten Schnittlinie A-A zeigt, verdeutlicht, dass nicht nur die Trokarhülse 3 kreisrund ist, sondern auch der schaftförmige Mittelteil 8 in diesem Bereich sowie das Arbeitsinstrument 2, das ebenfalls die Trokarhülse 3 durchsetzt. Bei der alternativen Ausführungsform gemäß Figur 4b ist der schaftförmige Mittelteil 8b oval ausgebildet und zwar derart, dass die in der Figur mit Y gekennzeichnete kurze Achse in der Versatzebene des Endoskopes 1 liegt. Diese wird in Figur 1 durch die Papierebene definiert. Deutlich sichtbar ist in Figur 1, dass sowohl der Distalteil 10 als auch der Handhabenteil 9 in derselben Ebene gegenüber der Längsachse 13 des schaftförmigen Mittelteils 8 abgebogen bzw. abgekröpft sind. Es wird deutlich, dass bei einer Schaftausbildung und -anordnung wie sie anhand von Figur 4b dargestellt ist, der nutzbare Freiraum innerhalb der Trokarhülse 3 deutlich größer ist als der bei einer runden Ausbildung wie es anhand von Figur 4a dargestellt ist.

### Bezugszeichenliste

- 1: - Endoskop
- 2: - Arbeitsinstrument
- 3: - Trokarhülse
- 4: - Endeffektoren
- 5, 6: - Griffteile
- 7: - Schaftteil
- 8: - schaftförmiger Mittelteil
- 8b: - schaftförmiger Mittelteil aus Figur 4b
- 9: - Handhabenteil
- 10: - Distalteil
- 11: - proximaler Schaftbereich
- 11b: - proximaler Schaftbereich in Figur 3b
- 12: - distaler Schaftbereich
- 12a: - distaler Schaftbereich in Figur 2a
- 12b: - distaler Schaftbereich in Figur 2b
- 13: - Längsachse von 8
- 14: - Optikfenster

## Patentansprüche

1. Starres Endoskop mit einem schaftförmigen Mittelteil (8), an den proximalseitig ein Handhabenteil (9) anschließt und der distalseitig ein Distalteil (10) mit einem Optikfenster (14) aufweist, bei dem der Handhabenteil (9) versetzt oder schräg zur Längsachse (13) des Mittelteils (8) angeordnet ist, **dadurch gekennzeichnet, dass** der Distalteil (10) versetzt oder schräg zur Längsachse (13) des Mittelteils (8) angeordnet ist.

2. Endoskop nach Anspruch 1, **dadurch gekennzeichnet, dass** Handhabenteil (9) und Distalteil (10) bezogen auf die Längsachse (13) des Mittelteils (8) fluchtend zueinander angeordnet sind.

3. Endoskop nach einem der vorhergehenden Ansprüche, **dadurch gekennzeichnet, dass** der Distalteil (10, 12a) bogenförmig ausgebildet ist.

4. Endoskop nach einem der vorhergehenden Ansprüche, **dadurch gekennzeichnet, dass** der Distalteil (10, 12, 12a, 12b) schaftförmig, den schaftförmigen Mittelteil (8) fortsetzend ausgebildet ist.

5. Endoskop nach einem der vorhergehenden Ansprüche, **dadurch gekennzeichnet, dass** am Handhabenteil (9)ein Optikanschluss und/oder einer Okular vorgesehen ist und dass das Optikfenster (14) über ein eingegliedertes optisches System mit dem Optikanschluss und/oder dem Okular optisch verbunden ist.

6. Endoskop nach einem der vorhergehenden Ansprüche, **dadurch gekennzeichnet, dass** das Distalteil (10) gegenüber dem Mittelteil (8) aufweitend ausgebildet ist und einen elektronischen Bildsensor aufweist, der optisch mit dem Optikfenster (14) verbunden ist und dessen elektrische Anschlüsse mittelbar oder unmittelbar zum Handhabenteil (9) geführt und dort herausgeführt sind.

7. Endoskop nach einem der vorhergehenden Ansprüche, **dadurch gekennzeichnet, dass** das Optikfenster (14) am distalen Ende des Distalteils (10), vorzugsweise schräg zur Längsachse des Distalteils (10) angeordnet ist.

8. Endoskop nach einem der vorhergehenden Ansprüche, **dadurch gekennzeichnet, dass** das Optikfenster (14) in einer Umfangswand des Distalteils (10, 12b) angeordnet ist.

9. Endoskop nach einem der vorhergehenden Ansprüche, **dadurch gekennzeichnet, dass** im Distalteil (10) oder im Handhabenteil (9) Beleuchtungsmittel vorgesehen sind, vorzugsweise mit mindestens einer Leuchtdiode arbeitende Beleuchtungsmittel.

10. Endoskop nach einem der vorhergehenden Ansprüche, **dadurch gekennzeichnet, dass** der Mittelteil (8b) einen ovalen Querschnitt aufweist, vorzugsweise derart, dass der Versatz oder die Schrägstellung des Distalteils (10) und/oder des Handhabenteils (9) in Richtung der kurzen Querschnittsachse (Y) angeordnet ist.
